# EUROPEAN PATENT APPLICATION

(11) **EP 1 266 660 A1**
(43) Date of publication of application: **18.12.2002**
(21) Application number: 01912450.2
(22) Date of filing: 16.03.2001
(51) Int. Cl.: A61K 31/455, A61P 43/00, A61P 39/00, A61P 25/00

(54) **HEME OXYGENASE-1 INDUCERS OR INDUCTION ENHANCERS**

(30) Priority: 17.03.2000 JP 2000076289
(71) Applicant: Tanaka, Toshio, Tsu-shi, Mie 514-8507 (JP)
(72) Inventor: Tanaka, Toshio, Tsu-shi, Mie 514-8507 (JP)
(74) Representative: VOSSIUS & PARTNER
(86) International application number: JP0102122
(87) International publication number: WO01068094

(57) **Abstract**

An agent for inducing heme oxygenase-1 or enhancing heme oxygenase-1 induction which comprises a nicotinamide derivative as an active ingredient is effective for pharmaceutical use because it promotes the remission of pathological conditions.

## Description

### TECHNICAL FIELD

The present invention relates to heme oxygenase-1 (HO-1) inducers or HO-1 induction enhancers.

### BACKGROUND ART

Heme oxygenase (HO) is the rate-limiting enzyme in heme catabolism, which is currently known to consist of three distinct isozymes: HO-1, HO-2 and HO-3. HO-1 is an isozyme whose expression is induced by a heme protein as a substrate for HO-1, a heavy metal, hypoxia and various oxidative stresses. In contrast, HO-2 is an isozyme expressed constitutively. HO-3 is a recently identified novel isozyme whose functions are currently unknown.

Among the three HO isozymes, HO-1 and HO-2 to say the least are expressed not only in the liver and the spleen, but also in other different cells or organs in the body, including neurons, vascular endothelial cells and smooth muscle cells. Further, the metabolites of HO pathway, biliverdin, free iron ion and carbon monoxide (CO), have various physiologically important effects.

In view of the foregoing, HO, particularly HO-1, is of interest in its contribution to various pathological physiologies in the circulatory system, the nerve system, etc. (see Japanese Journal of Circulation Research, Vol. 22, No. 2, 43-51, 1999).

Cerebral vasospasm is a specific pathological condition developed after aneurysmal subarachnoid hemorrhage (SAH) and is the most critical unfavorable factor for SAH patients. Cerebral vasospasm, which causes cerebral ischemia, is delayed contraction, a phenomenon where the narrowing of SAH-exposed large arteries at the base of the brain starts 4 to 14 days after the development of SAH. Recently, we have clarified that in cerebral arteries undergoing delayed contraction, HO-1 was highly induced in correspondence with the degree of contraction, and that HO-1 simultaneously served as an endogenous anti-contraction molecule to contribute to the spontaneous remission of cerebral vasospasm (see The Journal of Clinical Investigation, Vol. 104, No. 1, 59-66, July 1999).

This suggests that HO-1 induced upon the development of cerebral vasospasm may serve as a host defensive molecule. If there is an agent capable of inducing HO-1 or enhancing HO-1 induction during pathological conditions, such agent may be significantly effective for pharmaceutical use because it promotes the remission of pathological conditions. However, no such agent has been known previously.

1,2-Bis(nicotinamide)propane is known as a radical scavenger effective for vasospasm inhibition (see Japanese Patent Examined Publication No. 61-55911). However, this publication teaches no effect that results from HO-1 induction or HO-1 induction enhancement by the compound, nor does it provide any information on the remission of pathological conditions in various organs or cells where HO-1 is strongly induced during the pathological conditions, although it describes some effects of the compound as a radical scavenger.

### DISCLOSURE OF THE INVENTION

An object of the present invention is to provide an agent capable of inducing HO-1 or enhancing HO-1 induction. Another object of the present invention is to provide an agent capable of promoting the remission of a pathological condition by inducing more HO-1 or enhancing HO-1 induction in an organ or cells where HO-1 is strongly induced during the pathological condition; the agent does not substantially affect the normal body.

Our research efforts were directed to achieving the above objects, and we found that when a rat model of cerebral vasospasm was administered with 1,2-bis(nicotinamide)propane, the model showed significantly increased mRNA and protein expression levels of HO-1 induced in its basilar arterial wall cells, as compared to a non-administered model, whereby the remission of vasospasm was promoted. We have finally completed the invention based on this finding.

Namely, the present invention provides an agent for inducing heme oxygenase-1 or enhancing heme oxygenase-1. induction, which comprises a nicotinamide derivative as an active ingredient. The present invention also provides an agent for stimulating a host defense response or a cell damage defense response, which comprises a nicotinamide derivative as an active ingredient and stimulates the host defense response or the cell damage defense response by inducing heme oxygenase-1 or enhancing heme oxygenase-1 induction. Further, the present invention provides an agent for promoting the amelioration of a pathological condition, which comprises a nicotinamide derivative as an active ingredient and promotes the amelioration of a pathological condition in an organ or cells where heme oxygenase-1 is highly induced during the pathological condition. The present invention also provides an agent for promoting the amelioration of a pathological condition in an organ or cells of the circulatory system, the nerve system, etc. Furthermore, the present invention provides an agent for inhibiting vasospasm, which comprises a nicotinamide derivative as an active ingredient and inhibits vasospasm by inducing heme oxygenase-1 or enhancing heme oxygenase-1 induction. In particular, the present invention provides any one of the above agents wherein the nicotinamide derivative is 1,2-bis(nicotinamide)propane.

### BRIEF DESCRIPTION OF THE DRAWINGS

Figure 1 graphically shows expression levels of HO-1 mRNA in basilar arterial walls of rat cerebral vasospasm models as measured 2 days after intra-cisterna magna injection.
Figure 2 shows an SDS-PAGE pattern of HO-1 protein expressed in basilar arterial walls of rat cerebral vasospasm models as measured 2 days after intra-cisterna magna injection.
Figure 3 graphically shows the time course of basilar artery diameter in rat cerebral vasospasm models.

### BEST MODE FOR CARRYING OUT THE INVENTION

As used herein, nicotinamide derivatives include, for example, 1,2-bis(nicotinamide)propane and structurally similar compounds, with 1,2-bis(nicotinamide)propane being preferred. 1,2-Bis(nicotinamide)propane is a known compound and can be prepared as described in, for example, Japanese Patent Examined Publication No. 61-55911. The present invention also encompasses any other nicotinamide derivatives as long as they have the ability to induce HO-1 or enhance HO-1 induction. Such derivative can be prepared in a known manner with or without modifications.

The term "inducing heme oxygenase-1 or enhancing heme oxygenase-1 induction" or "inducing HO-1 or enhancing HO-1 induction" is intended to encompass the induction of HO-1 mRNA or protein expression or the enhancement of such induction.

As used herein, host defense responses or cell damage defense responses include, for example, *in vivo* responses for preventing a wide variety of cell damage which may occur in the body (e.g., cell damage caused by blood and ischemia) and *in vivo* responses for promoting the amelioration of pathological conditions.

Examples of the organ or cell where heme oxygenase-1 is highly induced during pathological conditions include those of the circulatory system and the nerve system, as well as any other organ or cell in the body, regardless of its anatomical and physiological classifications, as long as it can provide such high level induction of heme oxygenase-1. Specific examples include brain, liver, spleen, neurons, vascular endothelial cells, smooth muscle cells, cerebral arteries (e.g., basilar arteries) and glia cells.

The administration route and dose of the agent can be suitably determined according to the type of nicotinamide derivative to be used, the physique, age and physical condition of the patient, the type and severity of the disease to be treated, elapsed time after onset of the disease, etc. The agent is generally used in an amount of 0.01 to 3000 mg/day when parenterally administered (by intravenous, intramuscular, or subcutaneous route). In the case where 1,2-bis(nicotinamide)propane is used as an active ingredient, it is generally preferable to administer the agent continuously or intermittently by intravenous route.

The agent may be administered at any stage, including the period during which HO-1 will be induced in a host defensive manner in response to the progress of pathological condition. In the case of cerebral vasospasm, for example, administration within 2 weeks after onset of the disease is expected to provide a therapeutic effect.

The agent of the present invention is not only targeted to cerebral vasospasm developed from subarachnoid hemorrhage or the like, but also effective for other various diseases associated with a pathological environment where HO-1 is induced in the body as a host defensive molecule.

Dosage forms suitable for administration include, for example, solutions, suspensions, emulsions, tablets, capsules, granules, fine granules, powders and suppositories. Such dosage forms may be prepared in combination with pharmaceutically acceptable auxiliary materials such as appropriate liquid or solid excipients, fillers, extenders, solvents, emulsifiers, lubricants, flavoring agents, scents, dyes and buffers.

### EXAMPLES

The present invention will be further described in the following examples. The examples are provided for illustrative purposes only, and are not intended to limit the scope of the invention.

### Example 1

### Induction and induction enhancement of HO-1 mRNA expression by 1.2-bis(nicotinamide)propane in rat cerebral vasospasm model

The preparation of rat cerebral vasospasm model and the determination of HO-1 mRNA expression level were performed according to a documented method (see The Journal of Clinical Investigation, Vol. 104, No. 1, July 1999). That is, the cerebral vasospasm model was prepared by single injection of autologous arterial blood into the cisterna magna of a SD rat to create a single hemorrhage model, and the model thus prepared was used to study changes in mRNA expression level in basilar arterial walls caused by administration of 1,2-bis(nicotinamide)propane.

Each single hemorrhage model was injected with 0.5 ml autologous arterial blood into its cisterna magna. Immediately following the injection of autologous arterial blood, 1,2-bis(nicotinamide)propane was continuously administered for 2 days by intravenous route (0.2 ml/hr; 1 mg/kg/min). After administration, the level of mRNA expressed in basilar arterial walls was determined on day 2 using a quantitative RT-PCR technique.

In control experiments, the same procedure was repeated except that physiological saline was used instead of the above autologous arterial blood and/or the above 1,2-bis(nicotinamide)propane. The single hemorrhage model will present the strongest delayed vasospasm on day 2 after the injection of autologous arterial blood. It is for this reason that the mRNA expression level was determined on day 2.

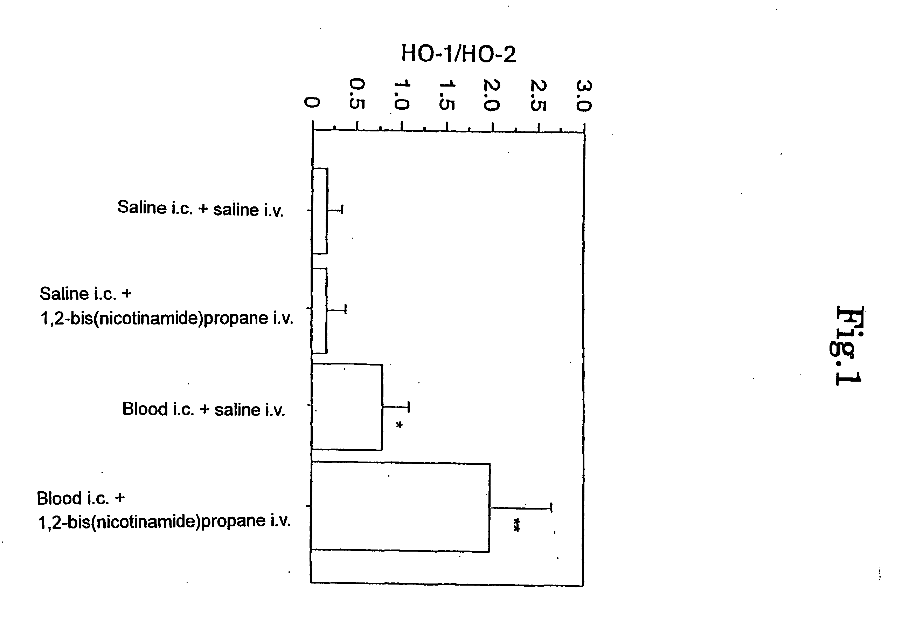

The results obtained are shown in Figure 1 as a ratio of HO-1 expression level to HO-2 expression level (n = 5 in each group). All data were expressed as mean ± SEM. The expression level of HO-2 was used as a denominator in calculating the ratio because the expression level of HO-2 was not assumed to change appreciably in this model and therefore, when used as a denominator, would enable changes in HO-1 expression level to be objectively evaluated while ignoring individual differences. In Figure 1, "HO-1/HO-2" represents the ratio of HO-1 expression level to HO-2 expression level, "saline i.c. + saline i.v." refers to a model injected with physiological saline into the cisterna magna and administered with physiological saline by intravenous route, "saline i.c. + 1,2-bis(nicotinamide)propane i.v." refers to a model injected with physiological saline into the cisterna magna and administered with 1,2-bis(nicotinamide)propane by intravenous route, "blood i.c. + saline i.v." refers to a model injected with autologous arterial blood into the cisterna magna and administered with physiological saline by intravenous route, and "blood i.c. + 1,2-bis(nicotinamide)propane i.v." refers to a model injected with autologous arterial blood into the cisterna magna and administered with 1,2-bis(nicotinamide)propane by intravenous route. The data were statistically processed according to variance analysis. A single asterisk (*) denotes a statistically significant difference from "saline i.c." at p<0.05 level and two asterisks (**) denote a statistically significant difference from "blood i.c. + saline i.v." at p<0.01 level.

Figure 1 indicates that the intravenous administration of 1,2-bis(nicotinamide)propane after autologous arterial blood injection results in a significant increase in HO-1 mRNA expression level.

### Example 2

### Induction and induction enhancement of HO-1 protein expression by 1,2-bis(nicotinamide)propane in rat cerebral vasospasm model

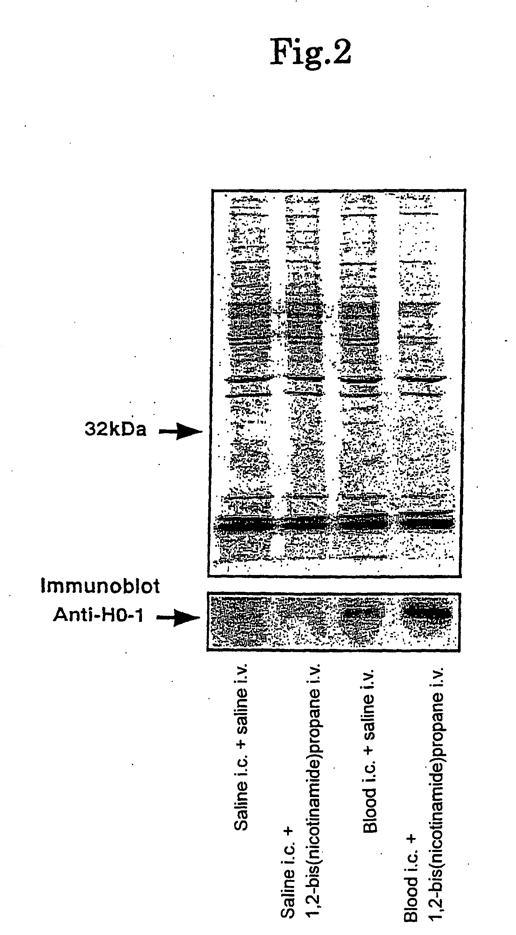

The same animal model as in Example 1 was used to study changes in HO-1 protein expression level after the same treatment as shown in Example 1. The changes in protein expression level were detected by Coomassie staining and Western blotting, as described in the above-mentioned reference. The results obtained are shown in Figure 2. In this figure, the upper and lower panels show the results of Coomassie staining and Western blotting, respectively. Each of the lanes shown in Figure 2 has the same definition as the corresponding sample in Figure 1.

Figure 2 indicates that 1,2-bis(nicotinamide)propane causes or enhances HO-1 protein induction after the injection of autologous arterial blood..

### Example 3

### 1,2-Bis(nicotinamide)propane-promoted amelioration of cerebral vasospasm in rat cerebral vasospasm model

The same model as in Example 1 was used to study changes over time in basilar artery diameter. The determination of basilar artery diameter was performed as described in the above-mentioned reference. That is, the diameter was measured angiographically on days 1 to 7 and expressed as a % change from the original diameter (% change before treatment was set to 0).

The model was injected with its autologous blood or physiological saline in the same manner as in Example 1 and then continuously administered with 1,2-bis(nicotinamide)propane or physiological saline by intravenous route for 7 consecutive days immediately after the injection.

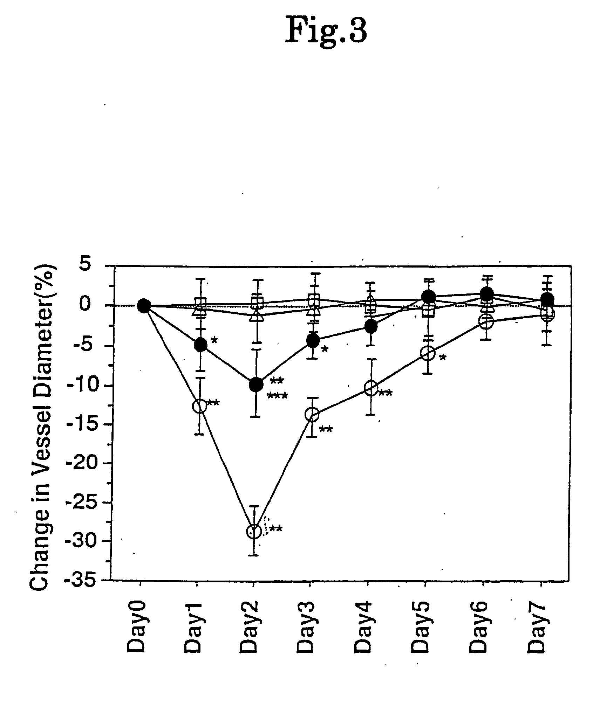

The results obtained are shown in Figure 3 (n = 3 in each group). All data were expressed as mean ± SEM. In this figure, the vertical axis represents the % change in blood vessel diameter. The horizontal axis represents the sequence of days (Day 0 to Day 7) after the injection of autologous arterial blood or physiological saline into the cisterna magna. The symbols in Figure 3 correspond to the respective models defined for Figure 1 in Example 1 as follows:
open triangle (Δ): saline i.c. + saline i.v.;
open square (□): saline i.c. + 1,2-bis(nicotinamide)-propane i.v.;
open circle (○): blood i.c. + saline i.v.; and
solid circle (●): blood i.c. + 1,2-bis(nicotinamide)-propane i.v.

The data was statistically processed such that intragroup comparison was performed by paired t-test and intergroup comparison was performed according to variance analysis. A single asterisk (*), two asterisks (**) and three asterisks (***) denote statistically significant differences from Day 0 at p<0.05 level, from Day 0 at p<0.01 level and from open circle (○) on Day 2 at p<0.01 level, respectively.

Figure 3 indicates that the administration of 1,2-bis(nicotinamide)propane results in a significant amelioration of delayed cerebral vasospasm. It also shows that the diameter of cerebral blood vessels is not affected in the absence of hemorrhage.

In view of the foregoing, 1,2-bis(nicotinamide)propane can induce and enhance the expression of HO-1 that serves as a host defensive molecule during pathological conditions, thereby promoting the remission of the pathological conditions. This compound is also expected to provide a HO-1 induction enhancer with fewer side effects because it does not affect the body in most cases excluding pathological conditions. This further suggests the possibility of producing a similar therapeutic effect in different pathological conditions other than cerebral vasospasm shown in the Examples, as long as those pathological conditions occur in organs or cells where HO-1 is induced.

### INDUSTRIAL APPLICABILITY

According to the present invention, nicotinamide derivatives are significantly effective for pharmaceutical use because they can cause or enhance HO-1 induction during pathological conditions to promote the remission of the pathological conditions. In particular, 1,2-bis(nicotinamide)propane causes or enhances HO-1 induction almost exclusively during pathological conditions, but does not substantially affect the normal body, thereby proving more effective for pharmaceutical use as a less toxic agent capable of promoting the remission of the pathological conditions.

## Claims

1. An agent for inducing heme oxygenase-1 or enhancing heme oxygenase-1 induction, which comprises a nicotinamide derivative as an active ingredient.

2. An agent for stimulating a host defense response or a cell damage defense response, which comprises a nicotinamide derivative as an active ingredient and stimulates the host defense response or the cell damage defense response by inducing heme oxygenase-1 or enhancing heme oxygenase-1 induction.

3. An agent for promoting the amelioration of a pathological condition, which comprises a nicotinamide derivative as an active ingredient and promotes the amelioration of a pathological condition in an organ or cells where heme oxygenase-1 is highly induced during the pathological condition.

4. The agent according to claim 3, wherein the organ or cells are those of the circulatory system or the nerve system.

5. An agent for inhibiting vasospasm, which comprises a nicotinamide derivative as an active ingredient and inhibits vasospasm by inducing heme oxygenase-1 or enhancing heme oxygenase-1 induction.

6. The agent according to any one of claims 1 to 5, wherein the nicotinamide derivative is 1,2-bis(nicotinamide)propane.
